# EUROPÄISCHE PATENTSCHRIFT

(11) **EP 0 424 722 B1**
(45) Veröffentlichungstag und Bekanntmachung des Hinweises auf die Patenterteilung: **06.07.1994**
(21) Anmeldenummer: 90119313.6
(22) Anmeldetag: 09.10.1990
(51) Int. Cl.: C07D 215/14, A61K 31/47

(54) **Substituierte (Chinolin-2-yl-methoxy)phenyl-thioharnstoffe**
Substituted (quinolin-2-yl-methoxy)phenyl-thioureas
(Quinolin-2-yl-méthoxy)phényl-thiourées substituées

(30) Priorität: 21.10.1989 DE 3935139
(43) Veröffentlichungstag der Anmeldung: 02.05.1991
(73) Patentinhaber: BAYER AG, 51368 Leverkusen (DE)
(72) Erfinder: Raddatz, Siegfried, Dr., W-5000 Köln 80 (DE); Mohrs, Klaus, Dr., W-5600 Wuppertal 1 (DE); Fugmann, Burkhard, Dr., W-5603 Wülfrath (DE); Fruchtmann, Romanis, W-5000 Köln 1 (DE); Kohlsdorfer, Christian, Dr., W-5042 Erftstadt (DE); Müller-Peddinghaus, Reiner, Prof.-Dr., W-5060 Bergisch Gladbach 2 (DE); Theisen-Popp, Pia, Dr., W-5060 Bergisch Gladbach 2 (DE)

(56) Entgegenhaltungen:
- GB-A- 2 202 223
- CHEMICAL ABSTRACTS, Band 97, 1982, Seite 715, Zusammenfassung Nr. 127519j, Columbus, Ohio, US

## Beschreibung

Die Erfindung betrifft substituierte (Chinolin-2-yl-methoxy)phenyl-thioharnstoffe, ein Verfahren zu ihrer Herstellung und ihre Verwendung in Arzneimitteln.

Es ist bekannt, daß Pyridyl- und Chinolinamine 5-Lipoxygenase-Inhibitoren sind [vgl. US 4.826.987]. Ferner werden in JP 82 64 675, Appl. 80/140 091 N,N-Dimethyl-N'-[3-(2-Chinolyl-methoxy)phenyl-harnstoffe beschrieben.

Es wurden nun substituierte (Chinolin-2-ylmethoxy)phenyl-thioharnstoffe der allgemeinen Formel (I) 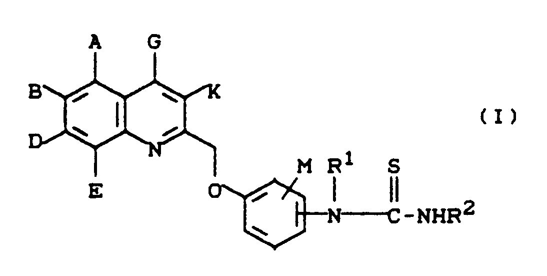
in welcher
A, B, D, E, G, K und M gleich oder verschieden sind und
- für Wasserstoff, Hydroxy, Halogen, Carboxy, Nitro, Trifluormethyl oder Trifluormethoxy stehen, oder
- für geradkettiges oder verzweigtes Alkyl, Alkoxy oder Alkoxycarbonyl mit jeweils bis zu 8 Kohlenstoffatomen stehen, oder
- für Aryl mit 6 bis 10 Kohlenstoffatomen stehen, das gegebenenfalls durch Halogen, Hydroxy, Nitro oder Cyano substituiert ist,

- R¹: - für Wasserstoff oder geradkettiges oder verzweigtes Alkyl mit bis zu 8 Kohlenstoffatomen steht, das gegebenenfalls durch Aryl mit 6 bis 10 Kohlenstoffatomen oder Cycloalkyl mit 3 bis 8 Kohlenstoffatomen substituiert ist, oder
- für Cycloalkyl mit 3 bis 8 Kohlenstoffatomen steht,
- R²: - für geradkettiges oder verzweigtes Alkyl mit bis zu 10 Kohlenstoffatomen steht, das gegebenenfalls durch Halogen oder Hydroxy substituiert ist, oder
- für Cycloalkyl mit 3 bis 8 Kohlenstoffatomen steht, das gegebenenfalls duch Halogen oder Cyano substituiert ist, oder
- für Aryl mit 6 bis 10 Kohlenstoffatomen steht, das gegebenenfalls bis zu 5-fach gleich oder verschieden durch Halogen, Nitro, Cyano, geradkettiges oder verzweigtes Alkyl mit bis zu 8 Kohlenstoffatomen oder durch eine Gruppe der Formel -SO₂-R³ substituiert ist,
worin
- R³: - geradkettiges oder verzweigtes Alkyl mit bis zu 8 Kohlenstoffatomen oder eine Gruppe der Formel -NR⁴R⁵ bedeutet,
worin
- R⁴ und R⁵: gleich oder verschieden sind und Wasserstoff, geradkettiges oder verzweigtes Alkyl mit bis zu 8 Kohlenstoffatomen oder Phenyl bedeuten,
oder
- R²: - für eine Gruppe der Formel -CO-R⁶ steht,
worin
- R⁶: - geradkettiges oder verzweigtes Alkyl mit bis zu 8 Kohlenstoffatomen oder Aryl mit 6 bis 10 Kohlenstoffatomen bedeutet, das gegebenenfalls bis zu 3-fach gleich oder verschieden durch Halogen, Nitro oder Cyano substituiert ist,
und deren Salze.

Im Rahmen der vorliegenden Erfindung werden physiologisch unbedenkliche Salze bevorzugt. Physiologisch unbedenkliche Salze der substituierten (Chinolin-2-yl-methoxy)phenyl-thioharnstoffe können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Bevorzugt sind Verbindungen der allgemeinen Formel (I)
in welcher
A, B, D, E, G, K und M gleich oder verschieden sind und
- für Wasserstoff, Hydroxy, Fluor, Chlor, Brom, Trifluormethyl, Trifluormethoxy oder für geradkettiges oder verzweigtes Alkyl oder Alkoxy mit jeweils bis zu 6 Kohlenstoffatomen stehen,
- für Phenyl stehen, das gegebenenfalls durch Fluor, Chlor oder Brom substituiert ist,

- R¹: - für Wasserstoff oder geradkettiges oder verzweigtes Alkyl mit bis zu 6 Kohlenstoffatomen steht, das gegebenenfalls durch Phenyl substituiert ist,
- R²: - für geradkettiges oder verzweigtes Alkyl mit bis zu 8 Kohlenstoffatomen steht, das gegebenenfalls durch Fluor, Chlor oder Brom substituiert ist, oder
- für Cyclopropyl, Cyclopentyl oder Cyclohexyl steht, das gegebenenfalls durch Fluor, Chlor oder Cyano substituiert ist, oder
- für Phenyl steht, das gegebenenfalls bis zu 4-fach gleich oder verschieden durch Fluor, Chlor, Brom, Nitro, Cyano, geradkettiges oder verzweigtes Alkyl mit bis zu 6 Kohlenstoffatomen oder durch eine Gruppe der Formel
   -SO₂-R³ substituiert ist,
worin
- R³: - geradkettiges oder verzweigtes Alkyl mit bis zu 6 Kohlenstoffatomen oder eine Gruppe der Formel -NR⁴R⁵ bedeutet,
worin
- R⁴ und R⁵: gleich oder verschieden sind und Wasserstoff, geradkettiges oder verzweigtes Alkyl mit bis zu 6 Kohlenstoffatomen oder Phenyl bedeuten,
oder
- R²: - für eine Gruppe der Formel -CO-R⁶ steht,
worin
- R⁶: - geradkettiges oder verzweigtes Alkyl mit bis zu 6 Kohlenstoffatomen oder Phenyl bedeutet, das gegebenenfalls bis zu 2-fach gleich oder verschieden durch Fluor, Chlor, Brom, Nitro oder Cyano substituiert ist,
und deren Salze.

Besonders bevorzugt sind Verbindungen der allgemeinen Formel (I),
in welcher
A, B, D, E, G, K und M gleich oder verschieden sind und
- für Wasserstoff, Fluor, Chlor, Brom, Trifluormethyl oder geradkettiges oder verzweigtes Alkyl mit bis zu 4 Kohlenstoffatomen stehen,

- R¹: - für Wasserstoff oder geradkettiges oder verzweigtes Alkyl mit bis zu 4 Kohlenstoffatomen steht,
- R²: - für geradkettiges oder verzweigtes Alkyl mit bis zu 6 Kohlenstoffatomen steht, oder
- für Cyclopentyl oder Cyclohexyl steht, das gegebenenfalls durch Cyano substituiert ist, oder
- für Phenyl steht, das gegebenenfalls bis zu 3-fach gleich oder verschieden durch Fluor, Chlor, Brom, Nitro, Cyano, geradkettiges oder verzweigtes Alkyl mit bis zu 4 Kohlenstoffatomen oder durch eine Gruppe der Formel -SO₂-R³ substituiert ist,
worin
- R³: - geradkettiges oder verzweigtes Alkyl mit bis zu 4 Kohlenstoffatomen oder eine Gruppe der Formel -NR⁴R⁵ bedeutet,
worin
- R⁴ und R⁵: gleich oder verschieden sind und Wasserstoff oder geradkettiges oder verzweigtes Alkyl mit bis zu 4 Kohlenstoffatomen bedeuten,
oder
- R²: - für eine Gruppe der Formel -CO-R⁶ steht,
worin
- R⁶: - Wasserstoff, geradkettiges oder verzweigtes Alkyl mit bis zu 4 Kohlenstoffatomen oder Phenyl bedeutet, das gegebenenfalls durch Fluor, Chlor oder Brom substituiert ist,
und deren Salze.

Ganz besonders bevorzugt sind Verbindungen der allgemeinen Formel (I), in denen die Chinolinmethoxygruppierung am Phenyl in 4-Stellung zur Thioharnstoffgruppe steht.

Außerdem wurde ein Verfahren zur Herstellung der erfindungsgemäßen Verbindungen der allgemeinen Formel (I) 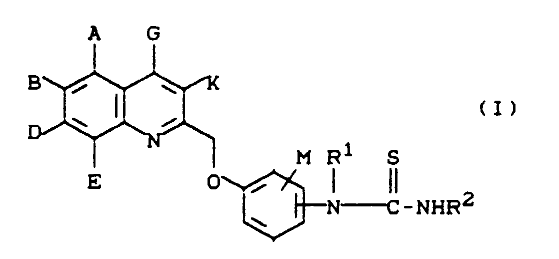
in welcher
A, B, D, E, G, K, M, R¹ und R² die oben angegebene Bedeutung haben,
gefunden, das dadurch gekennzeichnet ist, daß man Chinolylmethoxy-aniline der allgemeinen Formel (II) 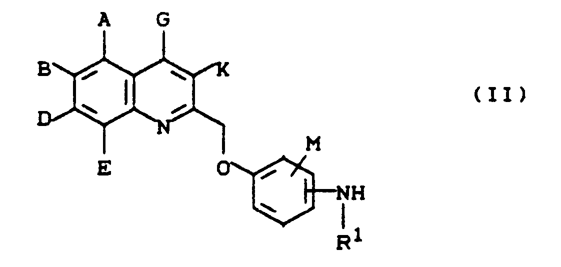
in welcher
A, B, D, E, G, K, M und R¹ die oben angegebene Bedeutung haben,
mit substituierten Isothiocyanaten der Formel (III)

S=C=N-R² (III)

in welcher
R² die oben angegebene Bedeutung hat,
in inerten Lösemitteln umsetzt.

Das erfindungsgemäße Verfahren kann durch folgendes Formelschema erläutert werden: 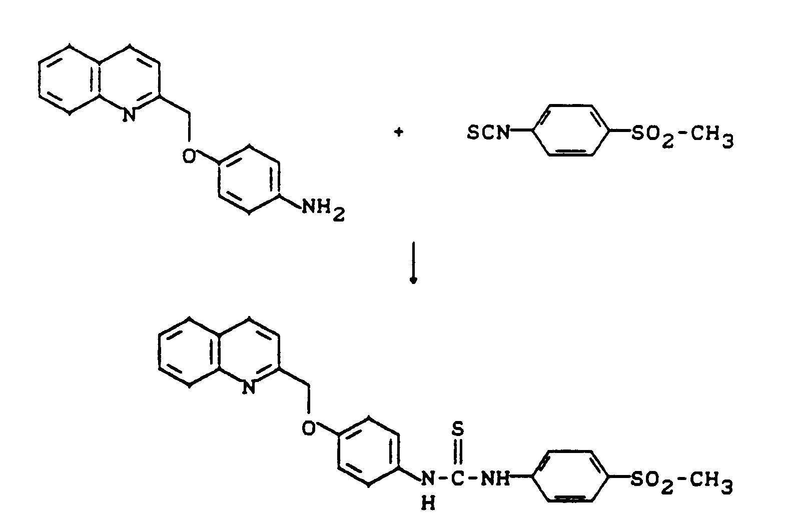
Als Lösemittel für die Umsetzung mit Isothiocyanaten (III) eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton, Nitromethan oder Nitroethan. Ebenso ist es möglich, Gemische der genannten Lösemittel zu verwenden. Besonders bevorzugt sind Nitromethan, Nitroethan und Dichlormethan.

Das Verfahren wird im allgemeinen in einem Temperaturbereich von 0°C bis +80°C, bevorzugt bei Raumtemperatur, durchgeführt.

Das Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Im allgemeinen setzt man 1 bis 3 Mol, bevorzugt 1 bis 2 Mol, besonders bevorzugt 1 Mol Isothiocyanat, bezogen auf 1 Mol des Amins, ein.

Die Amine der allgemeinen Formel (II) sind an sich bekannt oder können nach bekannten Methoden hergestellt werden [vgl. Ger. Offen. DE 36 07 382; US 4 826 987].

Die Isothiocyanate der allgemeinen Formel (III) sind an sich bekannt oder können nach üblicher Methode hergestellt werden [vgl. Beilstein 12, 453, Fieser 1, 844, 2, 323; Ann. Biochem. 67, 392 (1975); Indian J. Pharm. Sci. 48 (3), 53-59; Collect. Czech. Chem. Commun. 39 (1), 182-184; 38 (12), 3852-3856; J. Antibiot., 21 (8), 504-508 (Tokyo)].

Die erfindungsgemäßen substituierten (Chinolin-2-yl-methoxy)phenyl-thioharnstoffe können als Wirkstoffe in Arzneimitteln eingesetzt werden. Die Stoffe können als Hemmer von enzymatischen Reaktionen im Rahmen des Arachidonsäurestoffwechsels, insbesondere der Lipoxygenase, wirken.

Sie sind somit bevorzugt zur Behandlung und Verhütung von Erkrankungen der Atemwege wie Allergien/Asthma, Bronchitis, Emphysema, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen substituierten (Chinolin-2-yl-methoxy)phenyl-thioharnstoffe können sowohl in der Humanmedizin als auch in der Veterinärmedizin angewendet werden.

Die pharmakologischen Wirkdaten der erfindungsgemäßen Substanzen werden durch folgende Methode bestimmt:
Als Maß für die Lipoxygenase-Hemmung wurde die Freisetzung von Leukotrien B₄ (LTB₄) an polymorphkernigen Rattenleukozyten (PMN) nach Zugabe von Substanzen und Ca-Ionophor mittels reverse phase HPLC nach Borgeat, P. et al., Proc. Nat. Acad. Sci. 76, 2148 - 2152 (1979), bestimmt. Die in vivo-Aktivität der Verbindungen wurde mit dem Mäuseohr-Entzündungsmodell nach Young, J.M. et al., J. of Investigative Dermatology 82, 367 - 371, (1984) nachgewiesen.

In der Tabelle 1 sind beispielhaft die nach diesem Test erzielten Werte einiger erfindungsgemäßer Verbindungen aufgeführt:

**Tabelle 1**

| Beispiel | LO-Hemmung IC₅₀ (»M) |
|---|---|
| 3 | 0.036 |
| 4 | 0,033 |
| 5 | 0,41 |
| 6 | 0,36 |
| 7 | 0,24 |
| 8 | 0,29 |

Die neuen Wirkstoffe können in an sich bekannter Weise unter Verwendung inerter nichttoxischer, pharmazeutisch geeigneter Trägerstoffe oder Lösungsmittel in die üblichen Formulierungen überführt werden wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 10 bis 70 Gew.-%, in der Zubereitung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohole-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,01 bis 5 mg/kg Körpergewicht, zur Erzielung wirksamer Ergebnisse zu verabreichen. Bei oraler Applikation beträgt die Dosierung im allgemeinen etwa 0,1 bis 200 mg/kg, vorzugsweise 1 bis 100 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und zu dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen.

### Ausgangsverbindung

### Beispiel I

### 2-(4-Aminophenoxymethyl)chinolin

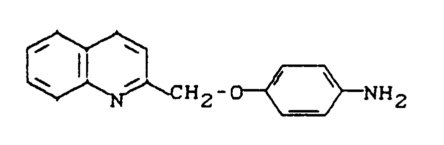

117,8 g (0,42 mol) 2-(4-Nitrophenoxymethyl)chinolin wurden in 1 l Methanol/Tetrahydrofuran (1:1) gelöst. Anschließend wurden ca. 5 g Raney-Nickel zugegeben und auf 35°C erwärmt. Dann wurden 63,1 g (1,26 mol) Hydrazinhydrat zugetropft und über Nacht gerührt. Es wurde vom Rückstand abfiltriert, die Lösung im Vakuum eingeengt und der Rückstand mit Methylenchlorid aufgenommen. Danach wurde mit Wasser gewaschen und die organische Phase mit konz. Salzsäure versetzt. Der ausgefallene Niederschlag wurde abfiltriert, mit 2 N Salzsäure gewaschen, in Wasser gelöst und mit 20%iger NaOH alkalisiert. Der Rückstand wurde im Vakuum getrocknet.
Ausbeute: 88,0 g (92,1% der Theorie)
Fp. = 131°C

### Herstellungsbeispiele (Formel I)

### Beispiel 1

### N-(4-Aminosulfonylphenyl)-N′-4-(chinolin-2-yl-methoxy) - phenyl-thioharnstoff

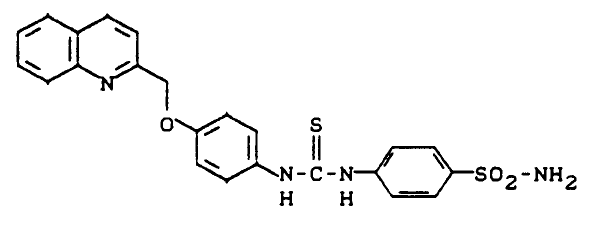

2,5 g (0,01 mol) der Verbindung aus Beispiel I wurden in 300 ml Nitroethan gelöst und mit 2,14 g (0,01 mol) 4-Aminosulfonylphenylisothiocyanat, gelöst in 300 ml Nitroethan, versetzt. Man läßt noch über Nacht bei Raumtemperatur rühren und filtriert dann den Niederschlag ab.
Ausbeute: 4,5 g (97,0% der Theorie) farblose Kristalle
Fp.: 211°C

### Beispiel 2

### N-(4-Mesylphenyl)-N′-4-(chinolin-2-yl-methoxy)phenylthioharnstoff

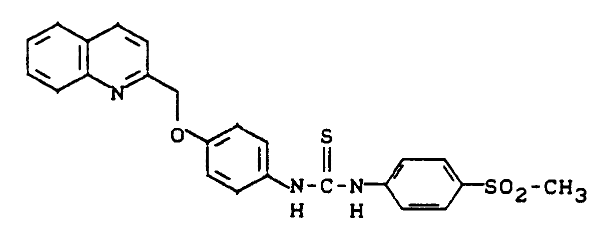

Die Titelverbindung wird in Analogie zu Beispiel 1 aus 2,5 g (0,01 mol) 2-(4-Aminophenoxymethyl)-chinolin und 2,13 g (0,01 mol) 4-Mesylphenylisothiocyanat in jeweils 100 ml Dichlormethan hergestellt.
Ausbeute: 4 g (86,3% der Theorie) farblose Kristalle
Fp.: 181°C (Zers.)

### Beispiel 3

### N-(2-Methyl-4-nitrophenyl)-N′-4-(chinolin2-yl-methoxy)phenyl-thioharnstoff

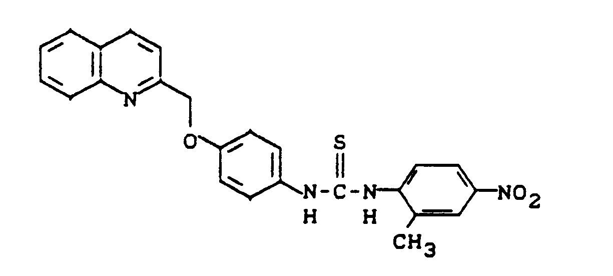

Die Titelverbindung wird in Analogie zu Beispiel 1 aus 2,5 g (0,01 mol) 2-(4-Aminophenoxymethyl)-chinolin und 1,94 g (0,01 mol) 2-Methyl-4-nitrophenylisothiocyanat in jeweils 50 ml Dichlormethan hergestellt.
Ausbeute: 3,6 g (81,1% der Theorie) gelbe Kristalle
Fp.: 154°C (Zers.)

### Beispiel 4

### N-(2-Methyl-4-nitro-5-chlorphenyl)-N′-4-(chinolin-2-yl-methyloxy)phenyl-thioharnstoff

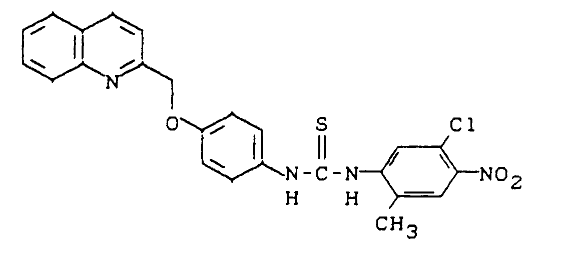

Die Titelverbindung wird in Analogie zu Beispiel 1 aus 2,5 g (0,01 mol) 2-(4-Aminophenoxymethyl)-chinolin und 2,3 g (0,01 mol) 2-Methyl-4-nitro-5-chlorphenylisothiocyanat in jeweils 100 ml Dichlormethan hergestellt.
Ausbeute: 3,8 g (79,4% der Theorie) gelbe Kristalle
Fp. : 169-170°C (Zers.) rekrist. aus Essigester/Petrolether

### Beispiel 5

### N-(2-Methyl-4,5-dichlorphenyl-N′-4-(chinolin-2-yl-methoxy)phenyl-thioharnstoff

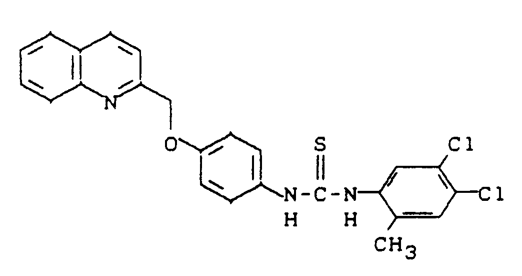

Die Titelverbindung wird in Analogie zu Beispiel 1 aus 4,6 g (0,0184 mol) 2-(4-Aminophenoxymethyl)-chinolin und 4 g (0,018 mol) 2-Methyl-4,5-dichlorphenylisothiocyanat in jeweils 100 ml Dichlormethan hergestellt.
Ausbeute: 7,4 g (87,8% der Theorie) farblose Kristalle
Fp.: 175-176°C

### Beispiel 6

### N-(4-Chlorbenzoyl)-N′-4-(chinolin-2-yl-methoxy)phenylthioharnstoff

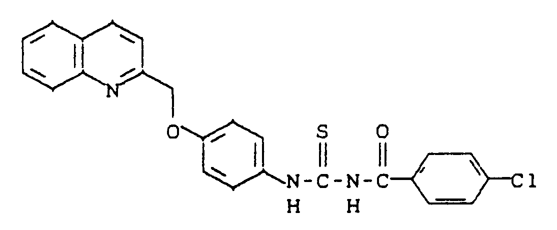

Die Titelverbindung wird in Analogie zu Beispiel 1 aus 5,67 g (0,0227 mol) 2-(4-Aminophenoxymethyl)-chinolin und 4,48 g (0,0227 mol) 4-Chlorbenzoylisothiocyanat in jeweils 100 ml Dichlormethan hergestellt.
Ausbeute: 6 g (59,3% der Theorie) hellgelbe Kristalle
Fp.: 193-194°C (Zers.)

### Beispiel 7

### N-(1-Cyano-cyclohexyl)-N′-4-(chinolin-2-yl-methoxy)phenyl-thioharnstoff

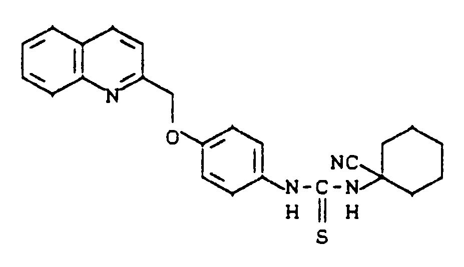

Die Titelverbindung wird in Analogie zu Beispiel 1 aus 3 g (0,012 mol) 2-(4-Amino-phenoxymethyl)-chinolin und 2 g (0,012 mol) 1-Cyano-1-isothiocyanatocyclohexan in jeweils 50 ml Dichlormethan hergestellt.
Ausbeute: 3,9 g (78,1% der Theorie) farblose Kristalle
Fp.: 213°C (Zers.).

### Beispiel 8

### N-(2-Methyl-4-chlorphenyl)-N′-4-(chinolin-2-yl-methoxy)phenyl-thioharnstoff

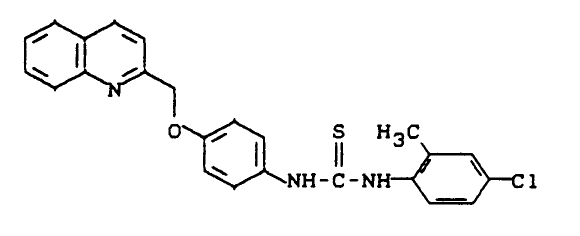

Die Titelverbindung wurde in Analogie zur Vorschrift der Beispiele 4 und 5 hergestellt.
Fp.: 148-150°C

## Patentansprüche (Patentansprüche für folgende(n) Vertragsstaat(en): AT, BE, CH, DE, DK, FR, GB, GR, IT, LI, LU, NL, SE)

1. Substituierte (Chinolin-2-yl-methoxy)phenyl-thioharnstoffe der allgemeinen Formel 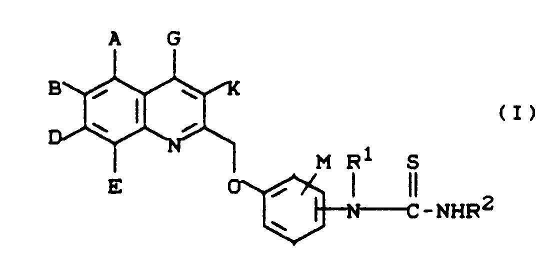 in welcher
A, B, D, E, G, K und M gleich oder verschieden sind und
- für Wasserstoff, Hydroxy, Halogen, Carboxy, Nitro, Trifluormethyl oder Trifluormethoxy stehen, oder
- für geradkettiges oder verzweigtes Alkyl, Alkoxy oder Alkoxycarbonyl mit jeweils bis zu 8 Kohlenstoffatomen stehen, oder
- für Aryl mit 6 bis 10 Kohlenstoffatomen stehen, das gegebenenfalls durch Halogen, Hydroxy, Nitro oder Cyano substituiert ist,
R¹
- für Wasserstoff oder geradkettiges oder verzweigtes Alkyl mit bis zu 8 Kohlenstoffatomen steht, das gegebenenfalls durch Aryl mit 6 bis 10 Kohlenstoffatomen oder Cycloalkyl mit 3 bis 8 Kohlenstoffatomen substituiert ist, oder
- für Cycloalkyl mit 3 bis 8 Kohlenstoffatomen steht,
R²
- für geradkettiges oder verzweigtes Alkyl mit bis zu 10 Kohlenstoffatomen steht, das gegebenenfalls durch Halogen oder Hydroxy substituiert ist, oder
- für Cycloalkyl mit 3 bis 8 Kohlenstoffatomen steht, das gegebenenfalls duch Halogen oder Cyano substituiert ist, oder
- für Aryl mit 6 bis 10 Kohlenstoffatomen steht, das gegebenenfalls bis zu 5-fach gleich oder verschieden durch Halogen, Nitro, Cyano, geradkettiges oder verzweigtes Alkyl mit bis zu 8 Kohlenstoffatomen oder durch eine Gruppe der Formel -SO₂-R³ substituiert ist,
worin
R³
- geradkettiges oder verzweigtes Alkyl mit bis zu 8 Kohlenstoffatomen oder eine Gruppe der Formel -NR⁴R⁵ bedeutet,
worin
R⁴ und R⁵ gleich oder verschieden sind und Wasserstoff, geradkettiges oder verzweigtes Alkyl mit bis zu 8 Kohlenstoffatomen oder Phenyl bedeuten,
oder
R²
- für eine Gruppe der Formel -CO-R⁶ steht,
worin
R⁶
- geradkettiges oder verzweigtes Alkyl mit bis zu 8 Kohlenstoffatomen oder Aryl mit 6 bis 10 Kohlenstoffatomen bedeutet, das gegebenenfalls bis zu 3-fach gleich oder verschieden durch Halogen, Nitro oder Cyano substituiert ist,
und deren Salze.

2. Substituierte (Chinolin-2-yl-methoxy)phenyl-thioharnstoffe der Formel (I) nach Anspruch 1
in welcher
in welcher
A, B, D, E, G, K und M gleich oder verschieden sind und
- für Wasserstoff, Hydroxy, Fluor, Chlor, Brom, Trifluormethyl, Trifluormethoxy oder für geradkettiges oder verzweigtes Alkyl oder Alkoxy mit jeweils bis zu 6 Kohlenstoffatomen stehen,
- für Phenyl stehen, das gegebenenfalls durch Fluor, Chlor oder Brom substituiert ist,
R¹
- für Wasserstoff oder geradkettiges oder verzweigtes Alkyl mit bis zu 6 Kohlenstoffatomen steht, das gegebenenfalls durch Phenyl substituiert ist,
R²
- für geradkettiges oder verzweigtes Alkyl mit bis zu 8 Kohlenstoffatomen steht, das gegebenenfalls durch Fluor, Chlor oder Brom substituiert ist, oder
- für Cyclopropyl, Cyclopentyl oder Cyclohexyl steht, das gegebenenfalls durch Fluor, Chlor oder Cyano substituiert ist, oder
- für Phenyl steht, das gegebenenfalls bis zu 4-fach gleich oder verschieden durch Fluor, Chlor, Brom, Nitro, Cyano, geradkettiges oder verzweigtes Alkyl mit bis zu 6 Kohlenstoffatomen oder durch eine Gruppe der Formel
-SO₂-R³ substituiert ist,
worin
R³
- geradkettiges oder verzweigtes Alkyl mit bis zu 6 Kohlenstoffatomen oder eine Gruppe der Formel -NR⁴R⁵ bedeutet,
worin
R⁴ und R⁵ gleich oder verschieden sind und Wasserstoff, geradkettiges oder verzweigtes Alkyl mit bis zu 6 Kohlenstoffatomen oder Phenyl bedeuten,
oder
R²
- für eine Gruppe der Formel -CO-R⁶ steht,
worin
R⁶
- geradkettiges oder verzweigtes Alkyl mit bis zu 6 Kohlenstoffatomen oder Phenyl bedeutet, das gegebenenfalls bis zu 2-fach gleich oder verschieden durch Fluor, Chlor, Brom, Nitro oder Cyano substituiert ist
und deren Salze.

3. Substituierte (Chinolin-2-yl-methoxy)phenyl-thioharnstoffe der Formel (I) nach Anspruch 1,
in welcher
A, B, D, E, G, K und M gleich oder verschieden sind und
- für Wasserstoff, Fluor, Chlor, Brom, Trifluormethyl oder geradkettiges oder verzweigtes Alkyl mit bis zu 4 Kohlenstoffatomen stehen,
R¹
- für Wasserstoff oder geradkettiges oder verzweigtes Alkyl mit bis zu 4 Kohlenstoffatomen steht,
R²
- für geradkettiges oder verzweigtes Alkyl mit bis zu 6 Kohlenstoffatomen steht, oder
- für Cyclopentyl oder Cyclohexyl steht, das gegebenenfalls durch Cyano substituiert ist, oder
- für Phenyl steht, das gegebenenfalls bis zu 3-fach gleich oder verschieden durch Fluor, Chlor, Brom, Nitro, Cyano, geradkettiges oder verzweigtes Alkyl mit bis zu 4 Kohlenstoffatomen oder durch eine Gruppe der Formel -SO₂-R³ substituiert ist,
worin
R³
- geradkettiges oder verzweigtes Alkyl mit bis zu 4 Kohlenstoffatomen oder eine Gruppe der Formel -NR⁴R⁵ bedeutet,
worin
R⁴ und R⁵ gleich oder verschieden sind und Wasserstoff oder geradkettiges oder verzweigtes Alkyl mit bis zu 4 Kohlenstoffatomen bedeuten,
oder
R²
- für eine Gruppe der Formel -CO-R⁶ steht,
worin
R⁶
- geradkettiges oder verzweigtes Alkyl mit bis zu 4 Kohlenstoffatomen oder Phenyl bedeutet, das gegebenenfalls durch Fluor, Chlor oder Brom substituiert ist,
und deren Salze.

4. Substituierte (Chinolin-2-yl-methoxy)phenyl-thioharnstoffe nach Anspruch 1 zur Bekämpfung von Krankheiten.

5. Verfahren zur Herstellung von substituierten (Chinolin-2-yl-methoxy)phenyl-thioharnstoffen der allgemeinen Formel 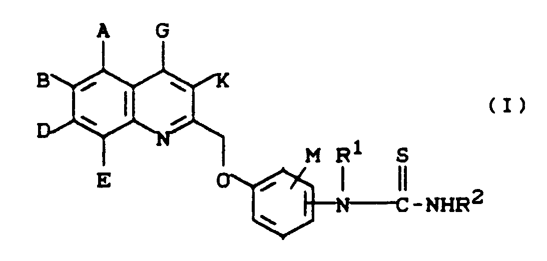 in welcher
A, B, D, E, G, K und M gleich oder verschieden sind und
- für Wasserstoff, Hydroxy, Halogen, Carboxy, Nitro, Trifluormethyl oder Trifluormethoxy stehen, oder
- für geradkettiges oder verzweigtes Alkyl, Alkoxy oder Alkoxycarbonyl mit jeweils bis zu 8 Kohlenstoffatomen stehen, oder
- für Aryl mit 6 bis 10 Kohlenstoffatomen stehen, das gegebenenfalls durch Halogen, Hydroxy, Nitro oder Cyano substituiert ist,
R¹
- für Wasserstoff oder geradkettiges oder verzweigtes Alkyl mit bis zu 8 Kohlenstoffatomen steht, das gegebenenfalls durch Aryl mit 6 bis 10 Kohlenstoffatomen oder Cycloalkyl mit 3 bis 8 Kohlenstoffatomen substituiert ist, oder
- für Cycloalkyl mit 3 bis 8 Kohlenstoffatomen steht,
R²
- für geradkettiges oder verzweigtes Alkyl mit bis zu 10 Kohlenstoffatomen steht, das gegebenenfalls durch Halogen oder Hydroxy substituiert ist, oder
- für Cycloalkyl mit 3 bis 8 Kohlenstoffatomen steht, das gegebenenfalls duch Halogen oder Cyano substituiert ist, oder
- für Aryl mit 6 bis 10 Kohlenstoffatomen steht, das gegebenenfalls bis zu 5-fach gleich oder verschieden durch Halogen, Nitro, Cyano, geradkettiges oder verzweigtes Alkyl mit bis zu 8 Kohlenstoffatomen oder durch eine Gruppe der Formel -SO₂-R³ substituiert ist,
worin
R³
- geradkettiges oder verzweigtes Alkyl mit bis zu 8 Kohlenstoffatomen oder eine Gruppe der Formel -NR⁴R⁵ bedeutet,
worin
R⁴ und R⁵ gleich oder verschieden sind und Wasserstoff, geradkettiges oder verzweigtes Alkyl mit bis zu 8 Kohlenstoffatomen oder Phenyl bedeuten,
oder
R²
- für eine Gruppe der Formel -CO-R⁶ steht,
worin
R⁶
- geradkettiges oder verzweigtes Alkyl mit bis zu 8 Kohlenstoffatomen oder Aryl mit 6 bis 10 Kohlenstoffatomen bedeutet, daß gegebenenfalls bis zu 3-fach gleich oder verschieden durch Halogen, Nitro oder Cyano substituiert ist,
und deren Salze, dadurch gekennzeichnet, das man Chinolylmethoxy-aniline der allgemeinen Formel (II) 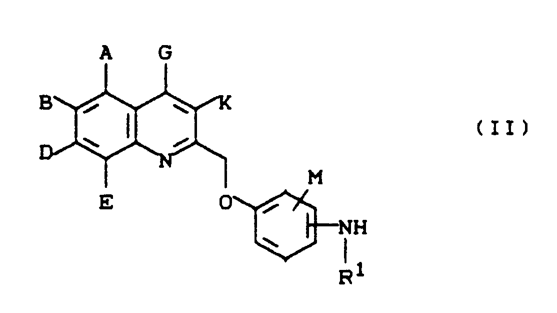 in welcher
A, B, D, E, G, K, M und R¹ die oben angegebene Bedeutung haben,
mit substituierten Isothiocyanaten der Formel (III)
S=C=N-R² (III)
in welcher
R² die oben angegebene Bedeutung hat,
in inerten Lösemitteln umsetzt.

6. Arzneimittel, enthaltend mindestens einen substituierten (Chinolin-2-yl-methoxy)phenyl-thioharnstoff nach Anspruch 1.

7. Verfahren zur Herstellung eines Arzneimittels nach Anspruch 6, dadurch gekennzeichnet, das man Verbindungen nach Anspruch 1 gegebenenfalls mit Hilfe von üblichen Hilfs- und Trägerstoffen in eine geeignete Applikationsform bringt.

8. Verwendung von substituierten (Chinolin-2-yl-methoxy)phenyl-thioharnstoffen nach Anspruch 1 zur Herstellung von Arzneimitteln.

9. Verwendung der substituierten (Chinolin-2-yl-methoxy)phenyl-thioharnstoffe nach Anspruch 1 zur Herstellung von Lipoxygenaseinhibitoren.

## Patentansprüche (Patentansprüche für folgende(n) Vertragsstaat(en): ES)

1. Verfahren zur Herstellung von substituierten (Chinolin-2-yl-methoxy)phenyl-thioharnstoffen der allgemeinen Formel 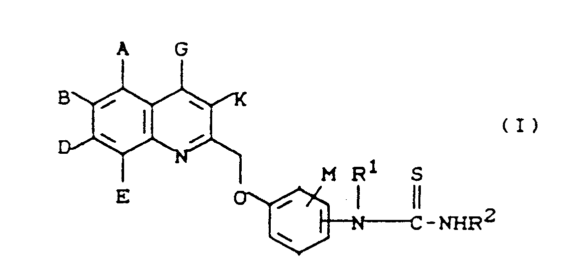 in welcher
A, B, D, E, G, K und M gleich oder verschieden sind und
- für Wasserstoff, Hydroxy, Halogen, Carboxy, Nitro, Trifluormethyl oder Trifluormethoxy stehen, oder
- für geradkettiges oder verzweigtes Alkyl, Alkoxy oder Alkoxycarbonyl mit jeweils bis zu 8 Kohlenstoffatomen stehen, oder
- für Aryl mit 6 bis 10 Kohlenstoffatomen stehen, das gegebenenfalls durch Halogen, Hydroxy, Nitro oder Cyano substituiert ist,
R¹
- für Wasserstoff oder geradkettiges oder verzweigtes Alkyl mit bis zu 8 Kohlenstoffatomen steht, das gegebenenfalls durch Aryl mit 6 bis 10 Kohlenstoffatomen oder Cycloalkyl mit 3 bis 8 Kohlenstoffatomen substituiert ist, oder
- für Cycloalkyl mit 3 bis 8 Kohlenstoffatomen steht,
R²
- für geradkettiges oder verzweigtes Alkyl mit bis zu 10 Kohlenstoffatomen steht, das gegebenenfalls durch Halogen oder Hydroxy substituiert ist, oder
- für Cycloalkyl mit 3 bis 8 Kohlenstoffatomen steht, das gegebenenfalls duch Halogen oder Cyano substituiert ist, oder
- für Aryl mit 6 bis 10 Kohlenstoffatomen steht, das gegebenenfalls bis zu 5-fach gleich oder verschieden durch Halogen, Nitro, Cyano, geradkettiges oder verzweigtes Alkyl mit bis zu 8 Kohlenstoffatomen oder durch eine Gruppe der Formel -SO₂-R³ substituiert ist,
worin
R³
- geradkettiges oder verzweigtes Alkyl mit bis zu 8 Kohlenstoffatomen oder eine Gruppe der Formel -NR⁴R⁵ bedeutet,
worin
R⁴ und R⁵ gleich oder verschieden sind und Wasserstoff, geradkettiges oder verzweigte: Alkyl mit bis zu 8 Kohlenstoffatomen oder Phenyl bedeuten,
oder
R²
- für eine Gruppe der Formel -CO-R⁶ steht,
worin
R⁶
- geradkettiges oder verzweigtes Alkyl mit bis zu 8 Kohlenstoffatomen oder Aryl mit 6 bis 10 Kohlenstoffatomen bedeutet, das gegebenenfalls bis zu 3-fach gleich oder verschieden durch Halogen, Nitro oder Cyano substituiert ist,
und deren Salze, dadurch gekennzeichnet, daß man Chinolylmethoxy-aniline der allgemeinen Formel (II) 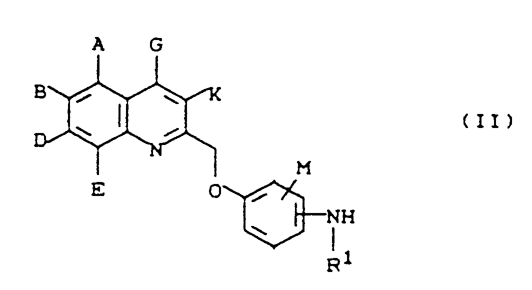 in welcher
A, B, D, E, G, K, M und R¹ die oben angegebene Bedeutung haben,
mit substituierten Isothiocyanaten der Formel (III)
S=C=N-R² (III)
in welcher
R² die oben angegebene Bedeutung hat,
in inerten Lösemitteln umsetzt.

## Claims (Claims for the following Contracting State(s): AT, BE, CH, DE, DK, FR, GB, GR, IT, LI, LU, NL, SE)

1. Substituted (quinolin-2-yl-methoxy)phenyl-thioureas of the general formula 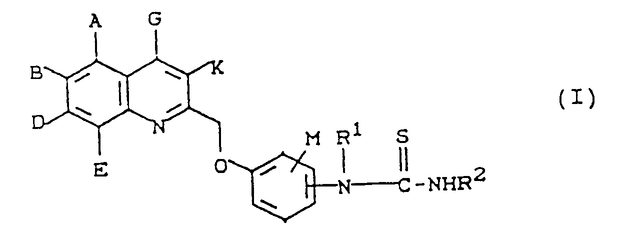 in which
A, B, D, E, G, K and M are identical or different and
- represent hydrogen, hydroxyl, halogen, carboxyl, nitro, trifluoromethyl or trifluoromethoxy, or
- represent straight-chain or branched alkyl, alkoxy or alkoxycarbonyl in each case having up to 8 carbon atoms, or
- represent aryl having 6 to 10 carbon atoms, which is optionally substituted by halogen, hydroxyl, nitro or cyano,
R¹
- represents hydrogen or straight-chain or branched alkyl having up to 8 carbon atoms, which is optionally substituted by aryl having 6 to 10 carbon atoms or cycloalkyl having 3 to 8 carbon atoms, or
- represents cycloalkyl having 3 to 8 carbon atoms,
R²
- represents straight-chain or branched alkyl having up to 10 carbon atoms, which is optionally substituted by halogen or hydroxyl, or
- represents cycloalkyl having 3 to 8 carbon atoms, which is optionally substituted by halogen or cyano, or
- represents aryl having 6 to 10 carbon atoms, which is optionally monosubstituted to pentasubstituted by identical or different substituents from the series comprising halogen, nitro, cyano, straight-chain or branched alkyl having up to 8 carbon atoms, or by a group of the formula -SO₂-R³,
in which
R³
- denotes straight-chain or branched alkyl having up to 8 carbon atoms or a group of the formula -NR⁴R⁵,
in which
R⁴ and R⁵ are identical or different and denote hydrogen, straight-chain or branched alkyl having up to 8 carbon atoms or phenyl,
or
R²
- represents a group of the formula -CO-R⁶,
in which
R⁶
- denotes straight-chain or branched alkyl having up to 8 carbon atoms or aryl having 6 to 10 carbon atoms, which is optionally monosubstituted to trisubstituted by identical or different substituents from the series comprising halogen, nitro or cyano,
and their salts.

2. Substituted (quinolin-2-yl-methoxy)phenyl-thioureas of the formula (I) according to Claim 1,
in which
A, B, D, E, G, K and M are identical or different and
- represent hydrogen, hydroxyl, fluorine, chlorine, bromine, trifluoromethyl, trifluoromethoxy or straight-chain or branched alkyl or alkoxy in each case having up to 6 carbon atoms,
- represent phenyl, which is optionally substituted by fluorine, chlorine or bromine,
R¹
- represents hydrogen or straight-chain or branched alkyl having up to 6 carbon atoms, which is optionally substituted by phenyl,
R²
- represents straight-chain or branched alkyl having up to 8 carbon atoms, which is optionally substituted by fluorine, chlorine or bromine, or
- represents cyclopropyl, cyclopentyl or cyclohexyl, which is optionally substituted by fluorine, chlorine or cyano, or
- represents phenyl which is optionally monosubstituted to tetrasubstituted by identical or different substituents from the series comprising fluorine, chlorine, bromine, nitro, cyano, straight-chain or branched alkyl having up to 6 carbon atoms, or by a group of the formula
-SO₂-R³,
in which
R³
- denotes straight-chain or branched alkyl having up to 6 carbon atoms or a group of the formula -NR⁴R⁵,
in which
R⁴ and R⁵ are identical or different and denote hydrogen, straight-chain or branched alkyl having up to 6 carbon atoms or phenyl,
or
R²
- represents a group of the formula -CO-R⁶,
in which
R⁶
- denotes straight-chain or branched alkyl having up to 6 carbon atoms or phenyl, which is optionally monosubstituted or disubstituted by identical or different substituents from the series comprising fluorine, chlorine, bromine, nitro or cyano,
and their salts.

3. Substituted (quinolin-2-yl-methoxy)phenyl-thioureas of the formula (I) according to Claim 1,
in which
A, B, D, E, G, K and M are identical or different and
- represent hydrogen, fluorine, chlorine, bromine, trifluoromethyl or straight-chain or branched alkyl having up to 4 carbon atoms,
R¹
- represents hydrogen or straight-chain or branched alkyl having up to 4 carbon atoms,
R²
- represents straight-chain or branched alkyl having up to 6 carbon atoms, or
- represents cyclopentyl or cyclohexyl, which is optionally substituted by cyano, or
- represents phenyl which is optionally monosubstituted to trisubstituted by identical or different substituents from the series comprising fluorine, chlorine, bromine, nitro, cyano, straight-chain or branched alkyl having up to 4 carbon atoms, or by a group of the formula -SO₂-R³,
in which
R³
- denotes straight-chain or branched alkyl having up to 4 carbon atoms or a group of the formula -NR⁴R⁵,
in which
R⁴ and R⁵ are identical or different and denote straight-chain or branched alkyl having up to 4 carbon atoms,
or
R²
- represents a group of the formula -CO-R⁶,
in which
R⁶
- denotes straight-chain or branched alkyl having up to 4 carbon atoms or phenyl, which is optionally substituted by fluorine, chlorine or bromine,
and their salts.

4. Substituted (quinolin-2-yl-methoxy)phenyl-thioureas according to Claim 1 for combating diseases.

5. Process for the preparation of substituted (quinolin-2-yl-methoxy)phenyl-thioureas of the general formula 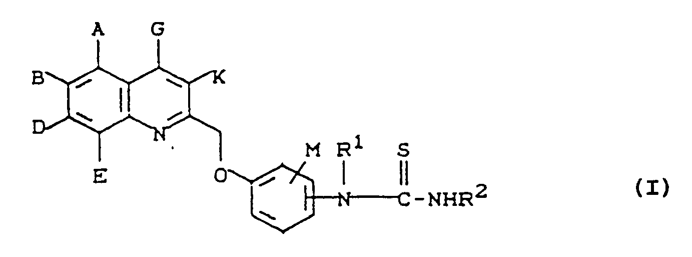 in which
A, B, D, E, G, K and M are identical or different and
- represent hydrogen, hydroxyl, halogen, carboxyl, nitro, trifluoromethyl or trifluoromethoxy, or
- represent straight-chain or branched alkyl, alkoxy or alkoxycarbonyl in each case having up to 8 carbon atoms, or
- represent aryl having 6 to 10 carbon atoms, which is optionally substituted by halogen, hydroxyl, nitro or cyano,
R¹
- represents hydrogen or straight-chain or branched alkyl having up to 8 carbon atoms, which is optionally substituted by aryl having 6 to 10 carbon atoms or cycloalkyl having 3 to 8 carbon atoms, or
- represents cycloalkyl having 3 to 8 carbon atoms,
R²
- represents straight-chain or branched alkyl having up to 10 carbon atoms, which is optionally substituted by halogen or hydroxyl, or
- represents cycloalkyl having 3 to 8 carbon atoms, which is optionally substituted by halogen or cyano, or
- represents aryl having 6 to 10 carbon atoms, which is optionally monosubstituted to pentasubstituted by identical or different substituents from the series comprising halogen, nitro, cyano, straight-chain or branched alkyl having up to 8 carbon atoms, or by a group of the formula -SO₂-R³,
in which
R³
- denotes straight-chain or branched alkyl having up to 8 carbon atoms or a group of the formula -NR⁴R⁵,
in which
R⁴ and R⁵ are identical or different and denote hydrogen, straight-chain or branched alkyl having up to 8 carbon atoms or phenyl,
or
R²
- represents a group of the formula -CO-R⁶,
in which
R⁶
- denotes straight-chain or branched alkyl having up to 8 carbon atoms or aryl having 6 to 10 carbon atoms, which is optionally monosubstituted to trisubstituted by identical or different substituents from the series comprising halogen, nitro or cyano,
and their salts, characterized in that quinolylmethoxy-anilines of the general formula (II) 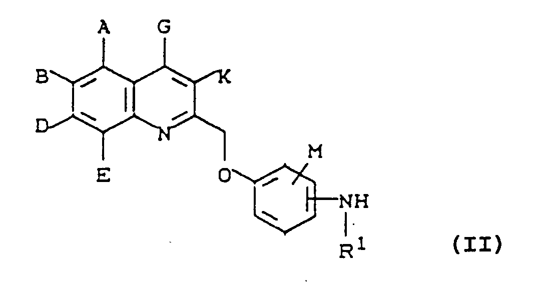 in which
A, B, D, E, G, K, M and R¹ have the abovementioned meaning,
are reacted with substituted isothiocyanates of the formula (III)
S=C=N-R² (III)
in which
R² has the abovementioned meaning,
in inert solvents.

6. Medicaments, containing at least one substituted (quinolin-2-yl-methoxy)phenyl-thiourea according to Claim 1.

7. Method for the production of a medicament according to Claim 6, characterized in that compounds according to Claim 1 are brought into a suitable form for administration, if appropriate with the aid of customary auxiliaries and excipients.

8. Use of substituted (quinolin-2-yl-methoxy)phenyl-thioureas according to Claim 1 for the production of medicaments.

9. Use of the substituted (quinolin-2-yl-methoxy)-phenyl-thioureas according to Claim 1 for the production of lipoxygenase inhibitors.

## Claims (Claims for the following Contracting State(s): ES)

1. Process for the preparation of substituted (quinolin-2-yl-methoxy)phenyl-thioureas of the general formula 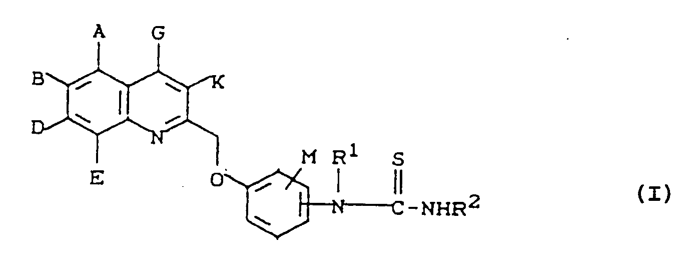 in which
A, B, D, E, G, K and M are identical or different and
- represent hydrogen, hydroxyl, halogen, carboxyl, nitro, trifluoromethyl or trifluoromethoxy, or
- represent straight-chain or branched alkyl, alkoxy or alkoxycarbonyl in each case having up to 8 carbon atoms, or
- represent aryl having 6 to 10 carbon atoms, which is optionally substituted by halogen, hydroxyl, nitro or cyano,
R¹
- represents hydrogen or straight-chain or branched alkyl having up to 8 carbon atoms, which is optionally substituted by aryl having 6 to 10 carbon atoms or cycloalkyl having 3 to 8 carbon atoms, or
- represents cycloalkyl having 3 to 8 carbon atoms,
R²
- represents straight-chain or branched alkyl having up to 10 carbon atoms, which is optionally substituted by halogen or hydroxyl, or
- represents cycloalkyl having 3 to 8 carbon atoms, which is optionally substituted by halogen or cyano, or
- represents aryl having 6 to 10 carbon atoms, which is optionally monosubstituted to pentasubstituted by identical or different substituents from the series comprising halogen, nitro, cyano, straight-chain or branched alkyl having up to 8 carbon atoms, or by a group of the formula -SO₂-R³,
in which
R³
- denotes straight-chain or branched alkyl having up to 8 carbon atoms or a group of the formula -NR⁴R⁵,
in which
R⁴ and R⁵ are identical or different and denote hydrogen, straight-chain or branched alkyl having up to 8 carbon atoms or phenyl,
or
R²
- represents a group of the formula -CO-R⁶,
in which
R⁶
- denotes straight-chain or branched alkyl having up to 8 carbon atoms or aryl having 6 to 10 carbon atoms, which is optionally monosubstituted to trisubstituted by identical or different substituents from the series comprising halogen, nitro or cyano,
and their salts, characterized in that quinolylmethoxy-anilines of the general formula (II) 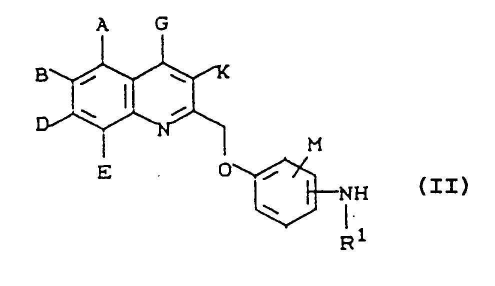 in which
A, B, D, E, G, K, M and R¹ have the abovementioned meaning,
are reacted with substituted isothiocyanates of the formula (III)
S=C=N-R² (III)
in which
R² has the abovementioned meaning,
in inert solvents.

## Revendications (Revendications pour l'(les) Etat(s) contractant(s) suivant(s): AT, BE, CH, DE, DK, FR, GB, GR, IT, LI, LU, NL, SE)

1. (Quinoléine-2-yl-méthoxy)phényl-thiourées substituées de formule générale 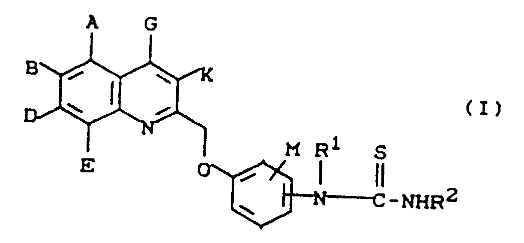 dans laquelle
A, B, D, E, G, K et M sont identiques ou différents et représentent
- de l'hydrogène, un groupe hydroxy, un halogène, un groupe carboxy, nitro, trifluorométhyle ou trifluorométhoxy, ou bien
- un groupe alkyle, alkoxy ou alkoxycarbonyle à chaîne droite ou ramifié ayant chacun jusqu'à 8 atomes de carbone, ou bien
- un groupe aryle de 6 à 10 atomes de carbone, qui est substitué le cas échéant par un halogène, un radical hydroxy, nitro ou cyano,
R¹
- représente de l'hydrogèe ou un groupe alkyle à chaîne droite ou ramifié ayant jusqu'à 8 atomes de carbone, qui est substitué le cas échéant par un radical aryle ayant 6 à 10 atomes de carbone ou cycloalkyle ayant 3 à 8 atomes de carbone, ou bien
- un groupe cycloalkyle de 3 à 8 atomes de carbone,
R²
- est un groupe alkyle à chaîne droite ou ramifié ayant jusqu'à 10 atomes de carbone, qui est substitué le cas échéant par un halogène ou un radical hydroxy, ou bien
- un groupe cycloalkyle de 3 à 8 atomes de carbone, qui est substitué le cas échéant par un halogène ou un radical cyano, ou bien
- un groupe aryle de 6 à 10 atomes de carbone qui est substitué le cas échéant jusqu'à 5 fois identiques ou différentes par un halogène, un radical nitro, cyano, alkyle à chaîne droite ou ramifié ayant jusqu'à 8 atomes de carbone ou par un groupe de formule -SO₂-R³,
dans laquelle
R³
- est un groupe alkyle à chaîne droite ou ramifié ayant jusqu'à 8 atomes de carbone ou un groupe de formule -NR⁴R⁵,
dans laquelle
R⁴ et R⁵ sont identiques ou différents et représentent de l'hydrogène, un groupe alkyle à chaîne droite ou ramifié ayant jusqu'à 8 atomes de carbone ou un groupe phényle,
ou bien
R²
- est un groupe de formule -CO-R⁶,
dans laquelle
R⁶
- est un groupe alkyle à chaîne droite ou ramifié ayant jusqu'à 8 atomes de carbone ou un groupe aryle de 6 à 10 atomes de carbone qui porte le cas échéant 1 à 3 substituants, identiques ou différents, halogéno, nitro ou cyano,
et leurs sels.

2. (Quinoléine-2-yl-méthoxy)phényl-thiourées substituées de formule (I) suivant la revendication 1,
dans laquelle
A, B, D, E, G, K et M sont identiques ou différents et représentent
- de l'hydrogène, un groupe hydroxy, du fluor, du chlore, du brome, un groupe trifluorométhyle, trifluorométhoxy ou un groupe alkyle ou alkoxy à chaîne droite ou ramifié ayant chacun jusqu'à 6 atomes de carbone,
- un groupe phényle qui est substitué le cas échéant par du fluor, du chlore ou du brome,
R¹
- représente de l'hydrogène ou un groupe alkyle à chaîne droite ou ramifié ayant jusqu'à 6 atomes de carbone, qui est éventuellement substitué par un radical phényle,
R²
- est un groupe alkyle à chaîne droite ou ramifié ayant jusqu'à 8 atomes de carbone, qui est substitué le cas échéant par du fluor, du chlore ou du brome, ou bien
- un groupe cyclopropyle, cyclopentyle ou cyclohexyle qui est substitué le cas échéant par du fluor, du chlore ou un radical cyano, ou bien
- un groupe phényle qui est substitué le cas échéant jusqu'à 4 fois fois identiques ou différentes par du fluor, du chlore, du brome, un radical nitro, cyano, alkyle à chaîne droite ou ramifié ayant jusqu'à 6 atomes de carbone ou par un groupe de formule -SO₂-R³,
dans laquelle
R³
- est un groupe alkyle à chaîne droite ou ramifié ayant jusqu'à 6 atomes de carbone ou un groupe de formule -NR⁴R⁵,
dans laquelle
R⁴ et R⁵ sont identiques ou différents et représentent de l'hydrogène, un groupe alkyle à chaîne droite ou ramifié ayant jusqu'à 6 atomes de carbone ou un groupe phényle,
ou bien
R²
- est un groupe de formule -CO-R⁶,
dans laquelle
R⁶
- est un groupe alkyle à chaîne droite ou ramifié ayant jusqu'à 6 atomes de carbone ou un groupe phényle qui est substitué le cas échéant jusqu'à 2 fois identiques ou différentes par du fluor, du chlore, du brome, un radical nitro ou cyano,
et leurs sels.

3. (Quinoléine-2-yl-méthoxy)phényl-thiourées substituées de formule (I) suivant la revendication 1,
dans laquelle
A, B, D, E, G, K et M sont identiques ou différents et représentent
- de l'hydrogène, du fluor, du chlore, du brome, un groupe trifluorométhyle ou un groupe alkyle à chaîne droite ou ramifié ayant jusqu'à 4 atomes de carbone,
R¹
- représente de l'hydrogène ou un groupe alkyle à chaîne droite ou ramifié ayant jusqu'à 4 atomes de carbone,
R²
- est un groupe alkyle à chaîne droite ou ramifié ayant jusqu'à 6 atomes de carbone, ou bien
- un groupe cyclopentyle ou cyclohexyle qui est substitué le cas échéant par un radical cyano, ou bien
- un groupe phényle qui est substitué le cas échéant jusqu'à 3 fois identiques ou différentes par du fluor, du chlore, du brome, un radical nitro, cyano, alkyle à chaîne droite ou ramifié ayant jusqu'à 4 atomes de carbone ou par un groupe de formule -SO₂-R³,
dans laquelle
R³
- est un groupe alkyle à chaîne droite ou ramifié ayant jusqu'à 4 atomes de carbone ou un groupe de formule -NR⁴R⁵,
dans laquelle
R⁴ et R⁵ sont identiques ou différents et représentent de l'hydrogène ou un groupe alkyle à chaîne droite ou ramifié ayant jusqu'à 4 atomes de carbone,
ou bien
R²
- est un groupe de formule -CO-R⁶,
dans laquelle
R⁶
- est un radical alkyle à chaîne droite ou ramifié ayant jusqu'à 4 atomes de carbone ou un radical phényle qui est substitué le cas échéant par du fluor, du chlore ou du brome,
et leurs sels.

4. (Quinoléine-2-yl-méthoxy)phényl-thiourées substituées suivant la revendication 1, destinées à combattre des maladies.

5. Procédé de production de (quinoléine-2-yl-méthoxy)phényl-thiourées substituées de formule générale 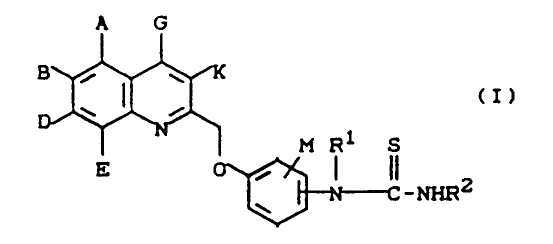 dans laquelle
A, B, D, E, G, K et M sont identiques ou différents et représentent
- de l'hydrogène, un groupe hydroxy, un halogène, un groupe carboxy, nitro, trifluorométhyle ou trifluorométhoxy, ou bien
- un groupe alkyle, alkoxy ou alkoxycarbonyle à chaîne droite ou ramifié ayant chacun jusqu'à 8 atomes de carbone, ou bien
- un groupe aryle de 6 à 10 atomes de carbone, qui est substitué le cas échéant par un halogène, un radical hydroxy, nitro ou cyano,
R¹
- représente de l'hydrogène ou un groupe alkyle à chaîne droite ou ramifié ayant jusqu'à 8 atomes de carbone, qui est substitué le cas échéant par un radical aryle ayant 6 à 10 atomes de carbone ou cycloalkyle ayant 3 à 8 atomes de carbone, ou bien
- un groupe cycloalkyle de 3 à 8 atomes de carbone,
R²
- est un groupe alkyle à chaîne droite ou ramifié ayant jusqu'à 10 atomes de carbone, qui est substitué le cas échéant par un halogène ou un radical hydroxy, ou bien
- un groupe cycloalkyle de 3 à 8 atomes de carbone, qui est substitué le cas échéant par un halogène ou un radical cyano, ou bien
- un groupe aryle de 6 à 10 atomes de carbone qui est substitué le cas échéant jusqu'à 5 fois identiques ou différentes par un halogène, un radical nitro, cyano, alkyle à chaîne droite ou ramifié ayant jusqu'à 8 atomes de carbone ou par un groupe de formule -SO₂-R³,
dans laquelle
R³
- est un groupe alkyle à chaîne droite ou ramifié ayant jusqu'à 8 atomes de carbone ou un groupe de formule -NR⁴R⁵,
dans laquelle
R⁴ et R⁵ sont identiques ou différents et **représentent de** l'hydrogène, un groupe alkyle à chaîne droite ou ramifié ayant jusqu'à 8 atomes de carbone ou un groupe phényle,
ou bien
R²
- est un groupe de formule -CO-R⁶,
dans laquelle
R⁶
- est un groupe alkyle à chaîne droite ou ramifié ayant jusqu'à 8 atomes de carbone ou un groupe aryle de 6 à 10 atomes de carbone qui porte le cas échéant 1 à 3 substituants, identiques ou différents, halogéno, nitro ou cyano,
et leurs sels,
caractérisé en ce qu'on fait réagir dans des solvants inertes des quinolylméthoxy-anilines de formule générale (II) 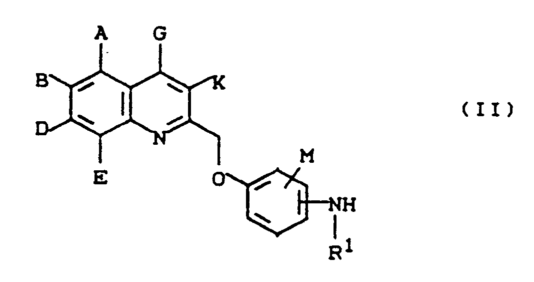 dans laquelle
A, B, D, E, G, K, M et R¹ ont la définition indiquée ci-dessus,
avec des isothiocyanates substitués de formule (III)
S=C=N-R² (III)
dans laquelle
R² a la définition indiquée ci-dessus.

6. Médicament contenant au moins une (quinoléine-2-yl-méthoxy)phényl-thiourée substituée suivant la revendication 1.

7. Procédé de préparation d'un médicament suivant la revendication 6, caractérisé en ce qu'on met sous une forme d'administration appropriée, éventuellement à l'aide de substances auxiliaires et de supports classiques, des composés suivant la revendication 1.

8. Utilisation de (quinoléine-2-yl-méthoxy)phényl-thiourées substituées suivant la revendication 1 pour la préparation de médicaments.

9. Utilisation des (quinoléine-2-yl-méthoxy)phényl-thiourées substituées suivant la revendication 1 pour la préparation d'inhibiteurs de lipoxygénase.

## Revendications (Revendications pour l'(les) Etat(s) contractant(s) suivant(s): ES)

1. Procédé de production de (quinoléine-2-yl-méthoxy)phényl-thiourées substituées de formule générale 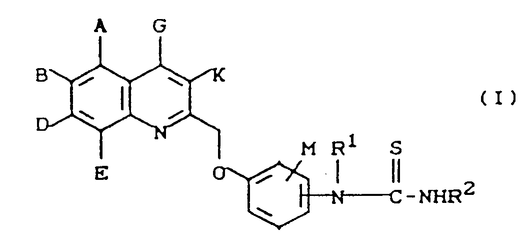 dans laquelle
A, B, D, E, G, K et M sont identiques ou différents et représentent
- de l'hydrogène, un groupe hydroxy, un halogène, un groupe carboxy, nitro, trifluorométhyle ou trifluorométhoxy, ou bien
- un groupe alkyle, alkoxy ou alkoxycarbonyle à chaîne droite ou ramifié ayant chacun jusqu'à 8 atomes de carbone, ou bien
- un groupe aryle de 6 à 10 atomes de carbone, qui est substitué le cas échéant par un halogène, un radical hydroxy, nitro ou cyano,
R¹
- représente de l'hydrogène ou un groupe alkyle à chaîne droite ou ramifié ayant jusqu'à 8 atomes de carbone, qui est substitué le cas échéant par un radical aryle ayant 6 à 10 atomes de carbone ou cycloalkyle ayant 3 à 8 atomes de carbone, ou bien
- un groupe cycloalkyle de 3 à 8 atomes de carbone,
R²
- est un groupe alkyle à chaîne droite ou ramifié ayant jusqu'à 10 atomes de carbone, qui est substitué le cas échéant par un halogène ou un radical hydroxy, ou bien
- un groupe cycloalkyle de 3 à 8 atomes de carbone, qui est substitué le cas échéant par un halogène ou un radical cyano, ou bien
- un groupe aryle de 6 à 10 atomes de carbone qui est substitué le cas échéant jusqu'à 5 fois identiques ou différentes par un halogène, un radical nitro, cyano, alkyle à chaîne droite ou ramifié ayant jusqu'à 8 atomes de carbone ou par un groupe de formule -SO₂-R³,
dans laquelle
R³
- est un groupe alkyle à chaîne droite ou ramifié ayant jusqu'à 8 atomes de carbone ou un groupe de formule -NR⁴R⁵,
dans laquelle
R⁴ et R⁵ sont identiques ou différents et représentent de l'hydrogène, un groupe alkyle à chaîne droite ou ramifié ayant jusqu'à 8 atomes de carbone ou un groupe phényle,
ou bien
R²
- est un groupe de formule -CO-R⁶,
dans laquelle
R⁶
- est un groupe alkyle à chaîne droite ou ramifié ayant jusqu'à 8 atomes de carbone ou un groupe aryle de 6 à 10 atomes de carbone qui porte le cas échéant 1 à 3 substituants, identiques ou différents, halogéno, nitro ou cyano,
et leurs sels, caractérisé en ce qu'on fait réagir dans des solvants inertes des quinolylméthoxy-anilines de formule générale (II) 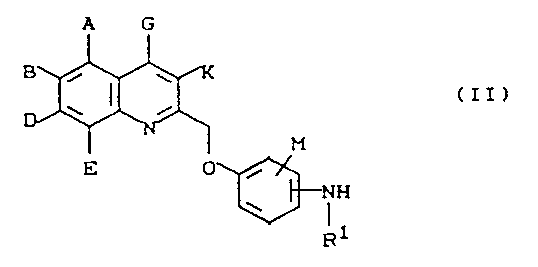 dans laquelle
A, B, D, E, G, K, M et R¹ ont la définition indiquée ci-dessus
avec des isothiocyanates substitués de formule (III)
S=C=N-R² (III)
dans laquelle
R² a la définition indiquée ci-dessus.
